# EUROPEAN PATENT APPLICATION

(11) **EP 4 797 293 A2**
(43) Date of publication of application: **26.08.2026**
(21) Application number: 26191485.7
(22) Date of filing: 24.12.2020
(51) Int. Cl.: H01F 7/121

(54) **PROPORTIONAL FLOW VALVE**

(30) Priority: 15.10.2020 CN 202011106344
(62) Document number(s) of the earlier application(s) in accordance with Art. 76 EPC: 20957539.8 / 4 201 462
(71) Applicant: Ambulanc(Shenzhen)Tech.Co.,Ltd., Shenzhen, Guangdong 518000 (CN)
(72) Inventor: GAO, Guifeng, Shenzhen, Guangdong, 518000 (CN); RAO, Qingchao, Shenzhen, Guangdong, 518000 (CN); WU, Yiming, Shenzhen, Guangdong, 518000 (CN); DENG, Xiaobin, Shenzhen, Guangdong, 518000 (CN); WANG, Ruiqiang, Shenzhen, Guangdong, 518000 (CN); HE, Yang, Shenzhen, Guangdong, 518000 (CN); CHEN, Wenfu, Shenzhen, Guangdong, 518000 (CN); YU, Liwei, Shenzhen, Guangdong, 518000 (CN)
(74) Representative: Herrero & Asociados, S.L.

(57) **Abstract**

The application disclose a proportional flow valve, comprising a valve house assembly, an electromagnet assembly, a valve core assembly, a sealing ring structure and a preload force structure. The valve housing assembly is formed a mounting cavity, an air inlet seat is formed at an opening end of the mounting cavity, and an air inlet passage extending up and down is formed in the air inlet seat; the electromagnet assembly is used for providing upward magnetic attraction; and the valve core assembly has an upward moving stroke driven by the magnetic attraction, an air-guide passage is formed in the valve core assembly, an inlet of the air-guide passage is formed at a lower end of the valve core assembly and is communicated with the air inlet passage, and an outlet is formed at side end of the valve core assembly.

## Description

### Cross-reference to related applications

This application claims the benefit of priority to China Patent Application No. 202011106344.2, filed Oct. 15, 2020, and entitled "Proportional flow valve", the entire content of which is incorporated herein by reference for all purposes.

### Technical field

The application relates to the technical field of gas flow control and valves, in particular to a proportional flow valve.

### Background

In the proportional flow valve, the flow output value has a linear relationship with the valve core opening, and the valve core opening has a linear relationship with the input electrical signal through the intermediate conversion mechanism, thus determining the linear relationship between the flow output value and the input signal.

Normally, regular solenoid valves adopt the structure of electromagnetic coil and linear spring, which is usually used as an on-off valve, that is, the electromagnetic coil overcomes the spring force to open the valve core after being energized. The electromagnetic force generated by the electromagnetic coil has an exponential relationship with the air gap, while the linear spring has a linear relationship with the compression or extension amount, in the case of same displacement, the added value of the electromagnetic force is greater than that of the linear spring, therefore the opening of the valve core can be directly opened to the maximum. However, it is difficult to meet the requirement that the proportional flow valve is stable in a certain position during the opening of the valve port, hence the linear relationship between the flow output value and input signal cannot be realized, and the proportional adjustment of the flow cannot be realized.

Patent EP 3 263 962 A1 proposes a valve for liquid and gas media, which comprises a valve body with a valve chamber. It can be in fluid communication with at least one inlet and at least one outlet. The valve features a precompression element that applies a precompression force to push the valve into its functioning position. The valve has a driver that works alongside it, allowing the driver to move the valve.

Patent DE 102 00 915 A1 provides an Electromagnetic valve for vehicle brake system. The purpose is to improve the working design and structure of non-pressure balance solenoid valve as a two-stage valve, which requires complex structure. The electromagnetic valve is hydraulically pressure balanced via a pressure balancing channel passing through the valve closure element and valve rod in the direction of the valve rod axis and extending into the magnet armature space. The armature space is connected only to the main brake cylinder connection via the pressure-balancing channel in the closed base position of the valve closure element.

In some working conditions, such as transfer ventilator, the venturi nozzle is usually used to introduce air after the proportional valve, but the gas passing through the proportional valve would generate a large back pressure when passing through the venturi nozzle, which leads to the regular proportional valve not working normally under the working condition of high back pressure and hence unable to realize the proportional adjustment of the flow.

So far, there is no proper proportional flow valve in the market that can solve the above problems. Therefore, it is necessary to design a new proportional flow valve which can not only solve the influence of high back pressure, but also work stably.

### Technical solutions

The main purpose of this application is to provide a proportional flow valve, which aims to solve the influence of high back pressure and allow the proportional flow valve work stably.

In order to achieve the above purpose, the proportional flow valve provided by the present application includes:
a valve housing assembly, formed with a mounting cavity with an opening at lower end, an air inlet seat is formed at an opening end of the mounting cavity and located at the middle part of the mounting cavity, and an air inlet passage extending up and down is formed in the air inlet seat;
an electromagnet assembly, arranged at an upper end of the mounting cavity and used for providing upward magnetic attraction;
a valve core assembly, arranged in the mounting cavity and located at the middle part of the mounting cavity, an annular mounting cavity is formed between the valve core assembly and an inner wall surface of the mounting cavity; driven by the magnetic attraction, the valve core assembly has an upward moving stroke; an air-guide passage is formed in the valve core assembly, an inlet of the air-guide passage is formed at a lower end of the valve core assembly and is communicated with the air inlet passage, and an outlet is formed at side end of the valve core assembly;
a sealing ring structure, including a sealing ring sealed in the annular mounting cavity, an inner ring of the sealing ring is sleeved on the valve core assembly, and an outer ring of the sealing ring is fixedly installed in the mounting cavity, so as to separate the annular mounting cavity into a closed upper annular mounting cavity and a closed lower annular mounting cavity, and the upper annular mounting cavity is communicated with the outlet of the air-guide passage;
a preload force structure, arranged between the valve core assembly and an inner wall of the mounting cavity, and used for providing downward preload force for the valve core assembly; and an air outlet gap communicating with the lower annular mounting cavity can be formed in use between the lower end of the valve core assembly and an upper end of the air inlet seat;
the electromagnet assembly includes a coil extending up and down and fixedly installed in the mounting cavity and a static armature fixedly installed in an inner hole of the coil, when the coil is electrified, the static armature forms the magnetic attraction; the valve core assembly includes a mobile armature and a valve core, the mobile armature is sandwiched between the static armature and the valve core, an upper end of the mobile armature is spaced from a lower end of the static armature, so as to form an electromagnet air gap; a lower end of the mobile armature is connected with an upper end of the valve core, and the air outlet gap is formed between a lower end face of the valve core and an upper end face of the air inlet seat;
the valve housing assembly includes:
   a housing, a lower end face of the housing is penetrated with a mounting hole extending up and down, and the coil is mounted in the mounting hole;
   a second annular support seat, installed at the lower end of the housing, and an inner hole of the second annular support seat is communicated with the mounting hole to jointly form the mounting cavity;
   a valve body, installed in the inner hole of the second annular support seat and located above the air inlet seat; and
   the valve body and the air inlet seat are integrally arranged; or
   the lower end face of the valve core is provided with a connecting hole;
   a lower end face of the mobile armature is provided with a third mounting hole extending up and down;
   the valve core assembly further includes a connecting column, an upper end of the connecting column is installed in the third mounting hole, a lower end of the connecting column is inserted into the connecting hole, and the air-guide passage is formed in the connecting column.

In an embodiment, the preload force structure includes an annular elastic sheet, an inner end of the annular elastic sheet is fixed to the valve core assembly, and an outer end is fixed to the inner wall of the mounting cavity; an annular surface of the annular elastic sheet is provided with a hollow pattern combination, the hollow pattern combination is a central symmetric structure and includes a plurality of hollow pattern units, and the number of the hollow pattern units is an integer multiple of natural number 2 or more.

In an embodiment, a first air-guide passage segment is through installed at the lower end face of the valve core, and the lower end face of the mobile armature is provided with a first mounting hole extending up and down; a sidewall surface of the first mounting hole is provided with a second air-guide passage segment, the upper end of the valve core is installed in the first mounting hole, and the first air-guide passage segment is communicated with the second air-guide passage segment to jointly form the air-guide passage; and/or
the mobile armature is positioned at a lower end of the inner hole of the coil, and a non-metallic magnetic separator is arranged between the upper end of the mobile armature and the lower end of the static armature; and/or
the mobile armature is at least partially positioned in the coil and has a mounting segment positioned in the coil;
the proportional flow valve further includes a first magnetic shield sleeve extending up and down, and the first magnetic shield sleeve is arranged between the coil and the mobile armature and at least partially covers the mounting segment.

In an embodiment, an upper end of the first magnetic shield sleeve is positioned in the inner hole of the coil, and a lower end of the first magnetic shield sleeve protrudes from a lower end of the coil;
the lower end of the static armature and the upper end of the mobile armature are both installed in the first magnetic shield sleeve, and the sidewall of the static armature is in sealing abutment with an inner wall of the first magnetic shield sleeve.

In an embodiment, the air-guide passage is formed in the valve core and the mobile armature; an air gap is formed between a sidewall of the mobile armature and an outer sidewall of the first magnetic shield sleeve, and the air gap communicates with the electromagnet air gap, the outlet of the air-guide passage and the upper annular mounting cavity.

In an embodiment, the mounting cavity is arranged in steps to form a first annular step surface arranged downwards;
the proportional flow valve further includes a first annular support seat located below the first annular step surface, and the outer ring of the first annular support seat is in sealing abutment with an inner sidewall of the mounting cavity, the inner ring of the first annular support seat is sleeved on the valve core assembly, and a first annular mounting space is formed between an upper end face of the first annular support seat and the first annular step surface, and the sealing ring is clamped in the annular space.

In an embodiment, an inner hole of the first annular support seat is set as a tapered hole with a small top and a large bottom;
the lower end of the valve core assembly extends from a lower end of the tapered hole and movably abuts against the upper end face of the air inlet seat, and the lower end of the valve core assembly is arranged in steps to form a second annular step surface arranged upwards;
the preload force structure includes an annular elastic sheet, an inner ring of the annular elastic sheet abuts against the second annular step surface, and an outer ring of the annular elastic sheet is fixed on a lower end face of the first annular support seat, and the second annular step surface is located above the lower end face of the first annular support seat.

In an embodiment, an upper end sidewall of the valve body is in threaded connection with an inner wall of the inner hole of the second annular support seat; and/or
the second annular support seat includes a support seat cylinder and a second magnetic shield sleeve extending upward from an upper end face of the support seat cylinder, the upper end face of the support seat cylinder is in sealing abutment with the lower end face of the housing, an upper end of the second magnetic shield sleeve extends into the inner hole of the coil, the lower end of the static armature and the upper end of the mobile armature are both installed in the second magnetic shield sleeve, and the side wall of the static armature is in sealing abutment with an inner wall of the second magnetic shield sleeve; and/or
an upper end face of the valve body is penetrated with a second mounting hole, and the second mounting hole is arranged in steps to form an abutment step surface arranged upward, and the proportional flow valve further includes a first clamping assembly, the first clamping assembly is annularly arranged and located on the abutment step surface; an outer ring of the first clamping assembly is in sealing abutment with an inner wall surface of the second mounting hole; a first annular clamping space is formed in the first clamping assembly; the inner ring of the sealing ring is hermetically sleeved on the valve core assembly, and the outer ring of the sealing ring is clamped in the first annular clamping space.

In an embodiment, the proportional flow valve further includes a transition block, the transition block is positioned in the mounting hole and below the coil, and the transition block is hermetically sleeved at a lower end of the second magnetic shield sleeve, a side wall of the transition block is in sealing abutment with a side wall of the mounting hole, and a lower end face of the transition block is mounted to the upper end face of the support seat cylinder; and/or
an inner hole of the support seat cylinder is set as a tapered hole with a small top and a large bottom, and the lower end of the valve core assembly is arranged in steps to form a third annular step surface arranged upwards, and the preload force structure includes an annular elastic sheet, the inner ring of the annular elastic sheet abuts against the third annular step surface, and the outer ring of the annular elastic sheet is fixed on the upper end face of the valve body.

In an embodiment, the connecting column is arranged in steps to form a fourth step surface arranged upwards, and a second annular mounting space is formed between the fourth step surface and the lower end face of the mobile armature;
the mounting cavity is formed with a fifth step surface arranged downwards, and the fifth step surface is located below the fourth step surface;
the preload force structure includes an annular elastic sheet, the inner ring of the annular elastic sheet is clamped in the second annular mounting space, and the outer ring of the annular elastic sheet abuts against the fifth step surface.

In an embodiment, the connecting column is also formed with a sixth step surface arranged downwards, and a third annular mounting space is formed between the sixth step surface and an upper end face of the valve core;
the mounting cavity is formed with a seventh annular step surface arranged downwards, and the seventh annular step surface is provided with a second clamping assembly, the second clamping assembly is annularly arranged, an outer ring of the second clamping assembly is in sealing abutment with the inner wall surface of the mounting cavity, and a fourth annular clamping space is formed in the second clamping assembly;
the inner ring of the sealing ring is clamped in the third annular mounting space, and the outer ring of the sealing ring is clamped in the fourth annular clamping space.

According the technical solution provided by the application, the air inlet passage is communicated with the air-guide passage, hence the gas at the air inlet of the air inlet passage is introduced into the closed upper annular mounting cavity, the sealing ring generates a downward pressure on the valve core assembly. And the preload force structure provides a downward preload force on the valve core assembly, when the proportional solenoid valve does not work, the pressure and preload force form a downward resultant force, so that the lower end of the valve core assembly is in sealing abutment with the upper end of the air inlet seat to avoid air leakage of the proportional solenoid valve; when the proportional solenoid valve works, the valve core assembly is subjected to an upward electromagnetic force provided by the electromagnet assembly, and the air pressure of the air inlet provides an upward thrust force for the valve core assembly; when there is no back pressure in the air outlet gap, the four forces of pressure, preload force, electromagnetic force and thrust force are balanced with each other, so that the valve core assembly can be stabilized at the working position; when there is back pressure in the air outlet gap, the back pressure provides an upward back pressure for the valve core, in this case, the five forces of pressure, preload force, electromagnetic force, thrust force and back pressure are balanced, so that the valve core can still be stable in the working position, therefore the proportional valve can work stably under different working conditions, the impact of high back pressure on the proportional flow valve is eliminated, and the effect is good.

### Brief description of the drawings

In order to illustrate the technical solutions in the embodiments of the present application or in the prior art more clearly, the drawings in the description of the embodiments of the present application will be briefly introduced below. It is obvious that the drawings in the following description only show some embodiments of the present application. A person of ordinary skill in the art can obtain other drawings according to the structures shown in these drawings without any creative effort.
Fig. 1 is a structural schematic diagram of a first embodiment of a proportional flow valve in accordance with the present application;
Fig. 2 is a structural schematic diagram of a second embodiment of a proportional flow valve in accordance with the present application;
Fig. 3 is a structural schematic diagram of a third embodiment of a proportional flow valve in accordance with the present application;
Fig. 4 is a structural diagram of an embodiment of the annular elastic sheet shown in Fig. 1;
Fig. 5 is a structural diagram of another embodiment of the annular elastic sheet shown in Fig. 1;
Fig. 6 is a curve diagram showing the relationship between electromagnetic force and electromagnet air gap of the proportional flow valve in accordance with the present application;
Fig. 7 is a curve diagram showing the relationship between preload force and valve core opening of the proportional flow valve in accordance with the present application;
Fig. 8 is a curve diagram showing the relationship between each force and displacement of the valve core of the proportional flow valve in accordance with the present application.

Reference signs in the drawings are as follows.

| Reference Sign | Feature | Reference Sign | Feature |
|---|---|---|---|
| 100 | Proportional flow valve | 351 | Fourth step surface |
| 1 | Valve housing assembly | 352 | Sixth step surface |
| 11 | First annular step surface | 41 | Sealing ring |
| 12 | Housing | 51 | Annular elastic sheet |
| 13 | Second annular support seat | 6 | Air inlet seat |
| 131 | Support seat cylinder | 61 | Air inlet passage |
| 132 | Second magnetic shield sleeve | 62 | Air outlet passage |
| 14 | Valve body | 7 | Non-metallic magnetic separator |
| 141 | Abutment step surface | 8 | First magnetic shield sleeve |
| 15 | Fifth step surface | 9 | First annular support seat |
| 16 | Seventh annular step surface | 10 | First clamping assembly |
| 2 | Electromagnet assembly | 101 | Transition block |
| 21 | Coil | 102 | Second clamping assembly |
| 22 | Static armature | a | Annular mounting cavity |
| 31 | Mobile armature | a1 | Upper annular mounting cavity |
| 311 | First mounting hole | a2 | Lower annular mounting cavity |
| 32 | Valve core | b | Air-guide passage |
| 33 | Second annular step surface | b1 | First air-guide passage segment |
| 34 | Third annular step surface | b2 | Second air-guide passage segment |
| 35 | Connecting column | | |

Features, functions, objects and advantages of the present application will be further illustrated with reference to the drawings and embodiments.

### Detailed description of disclosed embodiments

In the following, the technical solutions in the embodiments of the application will be clearly and completely described with reference to the drawings. It is apparent that the described embodiments are some but not all of the embodiments of the present application. All other embodiments obtained by a person of ordinary skill in the art without any creative effort on the basis of the embodiments in the present application shall fall into the scope of the present application.

It should be noted that the directional indications (such as up, down, left, right, front, back, etc.) in the embodiments of this application are only used to illustrate the relative position relationship among components and their movement in a certain point of view (as shown in the drawings), and if the point of view changes, the directional indications would also change accordingly.

In addition, terms such as "first" and "second" in the embodiments of this application are only used for descriptive purposes, and cannot be considered as indicating or implying their relative priorities or implicitly indicating the number of indicated technical features. Therefore, the features defined as "first" and "second" can explicitly or implicitly include at least one of the indicated features. In addition, the meaning of "and/or" in the description includes three alternative solutions. For example, "A and/or B" includes Solution A, Solution B, or Solution A and Solution B. Furthermore, the technical solutions of each embodiment may be combined with each other, on the premise that it can be achieved by those skilled in the art. When the combination of technical solutions is contradictory or impossible, it shall be considered that this combination of technical solutions does not exist and does not fall into the protection scope of the present application.

In the proportional flow valve, the flow output value has a linear relationship with the valve core opening, and the valve core opening has a linear relationship with the input electrical signal through the intermediate conversion mechanism, thus determining the linear relationship between the flow output value and the input signal.

Normally, regular solenoid valves adopt the structure of electromagnetic coil and linear spring, which is usually used as an on-off valve, that is, the electromagnetic coil overcomes the spring force to open the valve core after being energized. The electromagnetic force generated by the electromagnetic coil has an exponential relationship with the air gap, while the linear spring has a linear relationship with the compression or extension amount, in the case of same displacement, the added value of the electromagnetic force is greater than that of the linear spring, therefore the opening of the valve core can be directly opened to the maximum. However, it is difficult to meet the requirement that the proportional flow valve is stable in a certain position during the opening of the valve port, hence the linear relationship between the flow output value and input signal cannot be realized, and the proportional adjustment of the flow cannot be realized.

In some working conditions, such as transfer ventilator, the venturi nozzle is usually used to introduce air after the proportional valve, but the gas passing through the proportional valve would generate a large back pressure when passing through the venturi nozzle, which leads to the regular proportional valve not working normally under the working condition of high back pressure and hence unable to realize the proportional adjustment of the flow.

So far, there is no proper proportional flow valve in the market that can solve the above problems. Therefore, it is necessary to design a new proportional flow valve which can not only solve the influence of high back pressure, but also work stably.

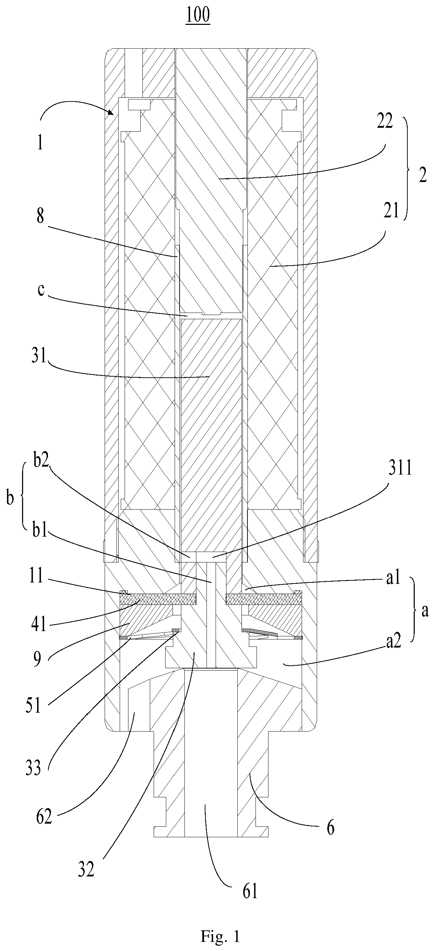

The application provides a proportional flow valve, wherein Figs. 1 to 5 are structural schematic diagrams of the proportional flow valve of embodiments in accordance with the application.

Referring to Figs. 1 to 3, the proportional flow valve 100 includes a valve housing assembly 1, an electromagnet assembly 2, a valve core assembly, a sealing ring 41 structure and a preload force structure. The valve housing assembly 1 is formed with a mounting cavity with an opening at lower end, an air inlet seat 6 is formed at an opening end of the mounting cavity and located at the middle part of the mounting cavity, and an air inlet passage 61 extending up and down is formed in the air inlet seat 6 ; the electromagnet assembly 2 is arranged at an upper end of the mounting cavity and used for providing upward magnetic attraction; the valve core assembly is arranged in the mounting cavity and located at the middle part of the mounting cavity, an annular mounting cavity a is formed between the valve core assembly and an inner wall surface of the mounting cavity; driven by the magnetic attraction, the valve core assembly has an upward moving stroke; an air-guide passage b is formed in the valve core assembly, an inlet of the air-guide passage b is formed at a lower end of the valve core assembly and is communicated with the air inlet passage 61 , and an outlet is formed at side end of the valve core assembly; the sealing ring structure includes a sealing ring 41 sealed in the annular mounting cavity a , an inner ring of the sealing ring 41 is sleeved on the valve core assembly, and an outer ring of the sealing ring 41 is fixedly installed in the mounting cavity, so as to separate the annular mounting cavity into a closed upper annular mounting cavity a1 and a closed lower annular mounting cavity a2, and the upper annular mounting cavity a1 is communicated with the outlet of the air-guide passage b; the preload force structure is arranged between the valve core assembly and an inner wall of the mounting cavity, and used for providing downward preload force for the valve core assembly; and an air outlet gap communicating with the lower annular mounting cavity a2 can be formed between the lower end of the valve core assembly and an upper end of the air inlet seat 6.

According the technical solution provided by the application, the air inlet passage 61 is communicated with the air-guide passage b, hence the gas at the air inlet of the air inlet passage 61 is introduced into the closed upper annular mounting cavity 41, the sealing ring 41 generates a downward pressure on the valve core assembly. And the preload force structure provides a downward preload force on the valve core assembly, when the proportional solenoid valve does not work, the pressure and preload force form a downward resultant force, so that the lower end of the valve core assembly is in sealing abutment with the upper end of the air inlet seat 6 to avoid air leakage of the proportional solenoid valve; when the proportional solenoid valve works, the valve core assembly is subjected to an upward electromagnetic force provided by the electromagnet assembly 2, and the air pressure of the air inlet provides an upward thrust force for the valve core assembly; when there is no back pressure in the air outlet gap, the four forces of pressure, preload force, electromagnetic force and thrust force are balanced with each other, so that the valve core assembly can be stabilized at the working position; when there is back pressure in the air outlet gap, the back pressure provides an upward back pressure for the valve core, in this case, the five forces of pressure, preload force, electromagnetic force, thrust force and back pressure are balanced, so that the valve core can still be stable in the working position, therefore the proportional valve can work stably under different working conditions, the impact of high back pressure on the proportional flow valve 100 is eliminated, and the effect is good.

It should be noted that the air inlet seat 6 is provided with an air outlet passage 62 extending up and down, when the valve core assembly moves up, the lower end of the valve core assembly and the upper end of the air inlet seat 6 form the air outlet gap, which communicates with the air inlet passage 61 and the air outlet passage 62 to realize air outlet.

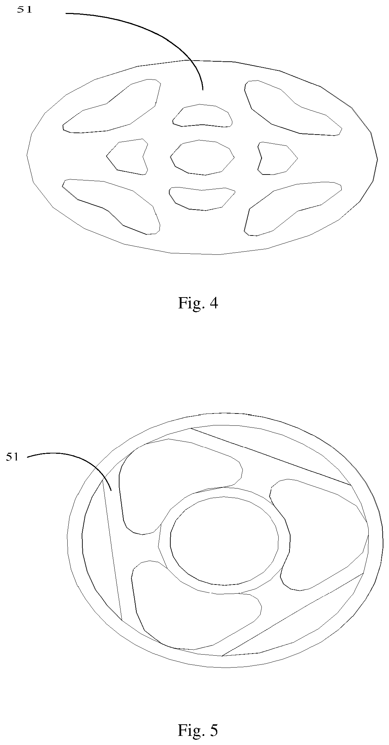

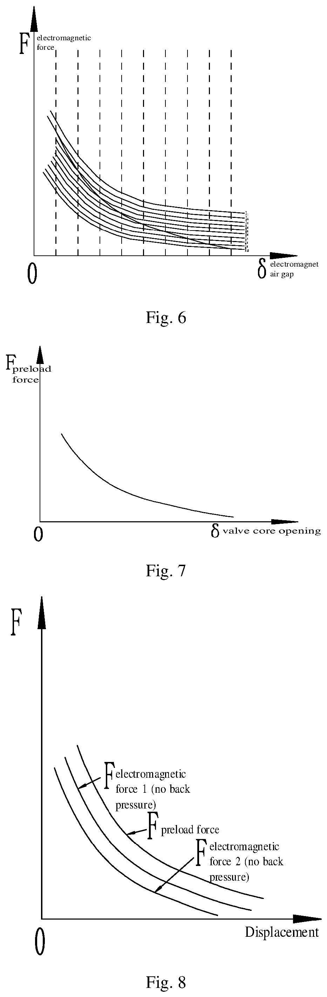

In order to enable the preload force structure provide preload force for the valve core assembly and realize proportional flow control, the electromagnetic force curve is determined on the electromagnetic force curve according to the opening ratio of the outlet air gap and the current change ratio. The electromagnetic force curve is nonlinear, so it is necessary to match the variation law of the preload force curve with the electromagnetic force curve to achieve an approximate linear relationship. Therefore, referring to Figs. 5 and 6, the preload force structure includes an annular elastic sheet 51, the inner end of annular elastic sheet 51 is fixed to the valve core assembly, and the outer end is fixed to the inner wall of the mounting cavity, so that the annular elastic sheet 51 would be prone to deform, thus facilitating the provision of preload force for the valve core assembly. The annular surface of the annular elastic sheet 51 is provided with a hollow pattern combination, the hollow pattern combination is a central symmetrical structure and includes a plurality of hollow pattern units, the number of the hollow pattern combination is an integer multiple of a natural number of 2 or more, so that the preload force structure can provide preload force for the valve core assembly, and the relationship curve between preload force and displacement is consistent with the electromagnetic force curve, thus facilitating the realization of proportional flow control.

The electromagnet assembly 2 includes a coil 21 extending up and down and fixedly installed in the mounting cavity and a static armature 22 fixedly installed in an inner hole of the coil 21, when the coil 21 is electrified, the static armature 22 forms the magnetic attraction; the valve core assembly includes a mobile armature 31 and a valve core 32, the mobile armature 31 is sandwiched between the static armature 22 and the valve core 33, an upper end of the mobile armature 31 is spaced from a lower end of the static armature 22, so as to form an electromagnet air gap c; a lower end of the mobile armature 31 is connected with an upper end of the valve core 32, and the air outlet gap is formed between a lower end face of the valve core 32 and an upper end face of the air inlet seat 6. In this way, because the lower end of the mobile art 31 is connected with the upper end of the valve core 32, the motion states of the mobile art 31 and the valve core 32 are consistent, when the coil 21 is energized, the static armature 22 forms magnetic attraction, by changing the size of the electromagnet air gap c, the magnetic attraction is changed, and the change amount of the electromagnet air gap c is also the moving distance of the valve core 32, and the magnetic attraction attracts the mobile armature 31 to move upwards, thereby driving the valve core 32 to move upwards, so that the outlet air gap is formed between the lower end face of the valve core 32 and the upper end face of the air inlet seat 6, hence the gas entering from the air inlet passage 61 can be discharged through the outlet air gap.

Specifically, the air-guide passage b is formed on the valve core 32 and mobile armature 31, so that the structure is simple and easy to manufacture.

In an embodiment, a first air-guide passage segment is through installed at the lower end face of the valve core 32, and the lower end face of the mobile armature 31 is provided with a first mounting hole 311 extending up and down; a sidewall surface of the first mounting hole 311 is provided with a second air-guide passage segment b2, the upper end of the valve core 32 is installed in the first mounting hole 311, and the first air-guide passage segment b1 is communicated with the second air-guide passage segment b2 to jointly form the air-guide passage b. With this arrangement, the outside air enters the first air-guide passage segment b1 and the second air-guide passage segment b2 from the air inlet passage 61 in turn, and finally converges in the closed upper annular mounting cavity a1. And the converged gas causes the sealing ring 41 to generate a downward pressure on the valve core 32, so that the lower end of the valve core 32 is in sealing abutment with the upper end of the air inlet seat 6, and the proportional flow valve 100 is prevented from leaking.

It should be noted that the air-guide passage b is formed on the valve core 32, so that the structure is simple and easy to manufacture.

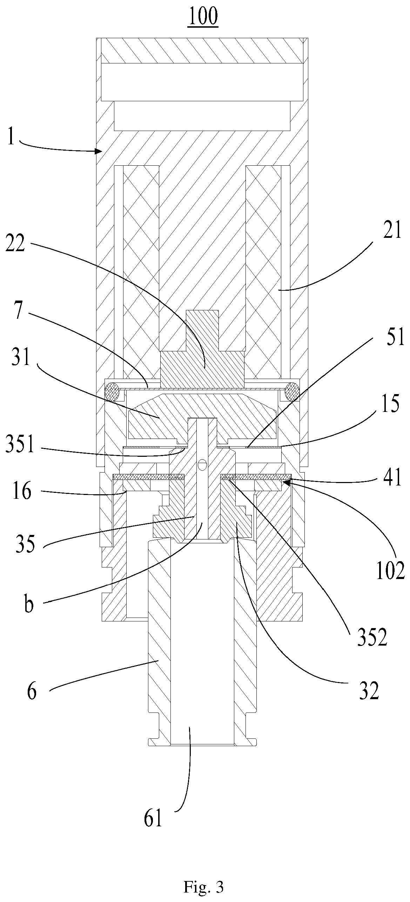

Referring to Fig. 3, the mobile armature 31 is positioned at a lower end of the inner hole of the coil 21, and a non-metallic magnetic separator 7 is arranged between the upper end of the mobile armature 31 and the lower end of the static armature 22. This arrangement plays a buffering role and prevents the mobile armature 31 from colliding with the static armature 22 to be damaged under the action of electromagnetic force.

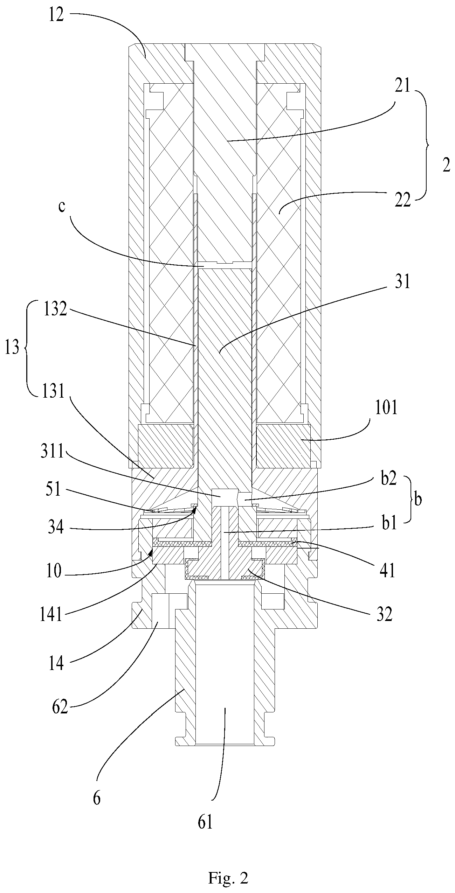

Referring to fig. 1 and fig. 2, the mobile armature 31 is at least partially positioned in the coil 21 and has a mounting segment positioned in the coil 21; the proportional flow valve 100 further includes a first magnetic shield sleeve 8 extending up and down, and the first magnetic shield sleeve 8 is arranged between the coil 21 and the mobile armature 31 and at least partially covers the mounting segment. In this way, the first magnetic shield sleeve 8 covers the mounting segment, so as to prevent the mobile armature 31 from generating electromagnetic force after the coil 21 is electrified, thereby avoiding affecting the sensitivity of the mobile armature 31 under the electromagnetic force of the static armature 22.

It should be noted that there are many types of materials can be used to achieve the magnetic isolation effect, such as silicon steel, stainless steel and copper. In the present application, the first magnetic shield sleeve 8 is made of copper.

In an embodiment, an upper end of the first magnetic shield sleeve 8 is positioned in the inner hole of the coil 21, and a lower end of the first magnetic shield sleeve 8 protrudes from a lower end of the coil 21;

the lower end of the static armature 22 and the upper end of the mobile armature 31 are both installed in the first magnetic shield sleeve 8, and the sidewall of the static armature 22 is in sealing abutment with an inner wall of the first magnetic shield sleeve 8. This arrangement prevents the static armature 22 and the first magnetic shield sleeve 8 from leaking air, thereby avoiding affecting the air tightness of the proportional flow valve 100.

Referring to Figs. 1 and 2, the air-guide passage b is formed in the valve core 32 and the mobile armature 31; an air gap is formed between a sidewall of the mobile armature 31 and an outer sidewall of the first magnetic shield sleeve 8, and because the air gap communicates with the electromagnet air gap c, the outlet of the air-guide passage b and the upper annular mounting cavity a1, the outside air can be converged in the closed upper annular mounting cavity a1 after passing through the electromagnet air gap c, the air-guide passage b and the air gap from the air inlet passage 61. The converged gas causes the sealing ring 41 to generate a downward pressure on the valve core 32, so that the lower end of the valve core 32 is in sealing abutment with the upper end of the air inlet seat 6, and the proportional flow valve 100 is prevented from leaking.

Referring to Fig. 1, the mounting cavity is arranged in steps to form a first annular step surface 11 arranged downwards; the proportional flow valve 100 further includes a first annular support seat 9 located below the first annular step surface 11, and the outer ring of the first annular support seat 9 is in sealing abutment with an inner sidewall of the mounting cavity, the inner ring of the first annular support seat 9 is sleeved on the valve core assembly, and a first annular mounting space is formed between an upper end face of the first annular support seat 9 and the first annular step surface 11, and the sealing ring 41 is clamped in the annular space. With this arrangement, the installation of the sealing ring 41 is realized.

In an embodiment, an inner hole of the first annular support seat 9 is set as a tapered hole with a small top and a large bottom; the lower end of the valve core assembly extends from a lower end of the tapered hole and movably abuts against the upper end face of the air inlet seat 6, and the lower end of the valve core assembly is arranged in steps to form a second annular step surface 33 arranged upwards; the preload force structure includes an annular elastic sheet 51, an inner ring of the annular elastic sheet 51 abuts against the second annular step surface 33, and an outer ring of the annular elastic sheet 51 is fixed on a lower end face of the first annular support seat 9, and because the second annular step surface 33 is located above the lower end face of the first annular support seat 9, the annular elastic sheet 51 generates a downward preload force on the valve core assembly, which has a good effect. And the tapered hole is arranged with a small top and a large bottom, thus providing an activity space for the annular elastic sheet 51.

Referring to Fig. 2, the valve housing assembly 1 includes a housing 12, a second annular support seat 13 and a valve body 14. A lower end face of the housing 12 is penetrated with a mounting hole extending up and down, and the coil 21 is mounted in the mounting hole; the second annular support seat is installed at the lower end of the housing 12, and an inner hole of the second annular support seat 13 is communicated with the mounting hole to jointly form the mounting cavity; the valve body is installed in the inner hole of the second annular support seat 13 and located above the air inlet seat 6; the valve body 14 and the air inlet seat 6 are integrally arranged, so as to facilitate precise machining.

Specifically, an upper end sidewall of the valve body 14 is in threaded connection with an inner wall of the inner hole of the second annular support seat 13. With this arrangement, the valve body 14 and the second annular support seat 13 are hermetically connected, and the disassembly and assembly are convenient.

Referring to Fig. 2, the second annular support seat 13 includes a support seat cylinder 131 and a second magnetic shield sleeve 132 extending upward from an upper end face of the support seat cylinder 131, the upper end face of the support seat cylinder 131 is in sealing abutment with the lower end face of the housing 12, an upper end of the second magnetic shield sleeve 132 extends into the inner hole of the coil 21, the lower end of the static armature 22 and the upper end of the mobile armature 31 are both installed in the second magnetic shield sleeve 132, and the side wall of the static armature 22 is in sealing abutment with an inner wall of the second magnetic shield sleeve 132. In this way, the upper end of the mobile armature 31 is covered by the second magnetic shield sleeve 132, so as to prevent the mobile armature 31 from generating electromagnetic force after the coil 21 is energized, thereby avoiding affecting the sensitivity of the mobile armature 31 under the electromagnetic force of the static armature 22.

In order to install the sealing ring 41, referring to Fig. 2, an upper end face of the valve body 14 is penetrated with a second mounting hole, and the second mounting hole is arranged in steps to form an abutment step surface 141 arranged upward, and the proportional flow valve 100 further includes a first clamping assembly 10, the first clamping assembly 10 is annularly arranged and located on the abutment step surface 141; an outer ring of the first clamping assembly 10 is in sealing abutment with an inner wall surface of the second mounting hole; a first annular clamping space is formed in the first clamping assembly 10; the inner ring of the sealing ring 41 is hermetically sleeved on the valve core assembly, and the outer ring of the sealing ring 41 is clamped in the first annular clamping space. With this arrangement, the installation of the sealing ring 41 is realized.

In order to assure the installation of the second annular support seat 13 more stable, the proportional flow valve 100 further includes a transition block 101, the transition block 101 is positioned in the mounting hole and below the coil 21 for supporting the coil 21, and the transition block 101 is hermetically sleeved at a lower end of the second magnetic shield sleeve 132, a side wall of the transition block 101 is in sealing abutment with a side wall of the mounting hole, and a lower end face of the transition block 101 is mounted to the upper end face of the support seat cylinder 131. In this way, the transition block 101 is connected with the support seat cylinder 131, so that the second annular support seat 13 can be installed more stably.

Referring to Fig. 2, an inner hole of the support seat cylinder 131 is set as a tapered hole with a small top and a large bottom, and the lower end of the valve core assembly is arranged in steps to form a third annular step surface 34 arranged upwards, and the preload force structure includes an annular elastic sheet 51, the inner ring of the annular elastic sheet 51 abuts against the third annular step surface 34, and the outer ring of the annular elastic sheet 51 is fixed on the upper end face of the valve body 14. In this way, the annular elastic sheet 51 generates downward preload force on the valve core assembly, which has a good effect. And the tapered hole is arranged with a small top and a large bottom, thus providing an activity space for the annular elastic sheet 51.

In order to prevent the transition block 101 from rotating relative to the support seat cylinder 131, an anti-rotation structure is further arranged between the lower end face of the transition block 101 and the upper end face of the support seat cylinder 131. In this way, the transition block 101 is prevented from rotating relative to the support seat cylinder 131, so that the air tightness of the proportional flow valve 100 is ensured and the effect is good.

It should be noted that the anti-rotation structure may be a convex and a groove which cooperate with each other. In the present application, the anti-rotation structure is a pin, through which the anti-rotation positioning of the transition block 101 and the support seat cylinder 131 is realized, and the transition block 101 is prevented from rotating relative to the support seat cylinder 131. Referring to Fig. 3, the lower end face of the valve core 32 is provided with a connecting hole; the lower end face of the mobile armature 31 is provided with a third mounting hole extending up and down;

the valve core assembly further includes a connecting column 35, an upper end of the connecting column 35 is installed in the third mounting hole, a lower end of the connecting column 35 is inserted into the connecting hole, and the air-guide passage b is formed in the connecting column 35. In this way, the valve core 32 and mobile armature 31 are connected through the connecting column 35, and the air-guide passage b is arranged at the connecting column 35, which has a simple structure and is easy to manufacture.

In an embodiment, the connecting column 35 is arranged in steps to form a fourth step surface 351 arranged upwards, and a second annular mounting space is formed between the fourth step surface 351 and the lower end face of the mobile armature 31; the mounting cavity is formed with a fifth step surface 15 arranged downwards, and the fifth step surface 15 is located below the fourth step surface 351; the preload force structure includes an annular elastic sheet 51, the inner ring of the annular elastic sheet 351 is clamped in the second annular mounting space, and the outer ring of the annular elastic sheet 51 abuts against the fifth step surface 15. With this arrangement, the installation of the annular elastic sheet 51 is realized, and the annular elastic sheet 51 exerts a preload force on the valve core 32 at the initial position, so that the sealing effect of the proportional flow valve 100 is good.

It should be noted that the inner ring of the annular elastic sheet 51 is clamped in the second annular mounting space, and the inner ring of the annular elastic sheet 51 can move up and down in the second annular mounting space. Specifically, referring to Fig. 3, the inner ring of the annular elastic sheet 51 abuts on the fourth step surface 351, which facilitates the activity of the annular elastic sheet 51 and has a good effect.

In an embodiment, the connecting column 35 is also formed with a sixth step surface 352 arranged downwards, and a third annular mounting space is formed between the sixth step surface 352 and an upper end face of the valve core 32; the mounting cavity is formed with a seventh annular step surface 16 arranged downwards, and the seventh annular step surface 16 is provided with a second clamping assembly 102, the second clamping assembly 102 is annularly arranged, an outer ring of the second clamping assembly 102 is in sealing abutment with the inner wall surface of the mounting cavity, and a fourth annular clamping space is formed in the second clamping assembly 102; the inner ring of the sealing ring 41 is clamped in the third annular mounting space, and the outer ring of the sealing ring 41 is clamped in the fourth annular clamping space. With this arrangement, the installation of the sealing ring 41 is realized with good effect.

It should be noted that in the present application, the air gap refers to the valve core opening of the proportional flow valve, and the gap formed by the gap between the lower end of the static armature and the end of the mobile armature is an electromagnet air gap. By changing the electromagnet air gap, the electromagnetic force is changed. And the air gap variation is also the valve core variation. Referring to Figs. 6 to 8, the electromagnetic force is inversely quadratic proportional to the air gap variation (equal to the valve opening), and the preload force is also inversely quadratic proportional to the valve opening, the variation law of the electromagnetic force curve and the preload force curve is the same, and the distance between the electromagnetic force curve and the preload force curve is always the same, so that the force for realizing the valve core balance together with the electromagnetic force and preload force is a constant value. And by adjusting the distance between the electromagnetic force curve and preload force curve, the proportional flow valve of the application may be applied to the working conditions with and without back pressure, and the effect is good.

The above are only preferred embodiments of the present application and are not intended to limit the present application. Any equivalent structural transformation made by using the contents of the specification and drawings of this application under the concept of this application, or direct/indirect application in other related technical fields are included in the patent protection scope of this application.

## Claims

1. A proportional flow valve (100), comprising:
a valve housing assembly (1), formed with a mounting cavity with an opening at a lower end, an air inlet seat (6) is formed at an opening end of the mounting cavity and located at the middle part of the mounting cavity, and an air inlet passage (61) extending up and down is formed in the air inlet seat (6);
an electromagnet assembly (2), arranged at an upper end of the mounting cavity and used for providing upward magnetic attraction;
a valve core assembly, arranged in the mounting cavity and located at the middle part of the mounting cavity, an annular mounting cavity (a) is formed between the valve core assembly and an inner wall surface of the mounting cavity; driven by the magnetic attraction, the valve core assembly has an upward moving stroke; an air-guide passage (b) is formed in the valve core assembly, an inlet of the air-guide passage (b) is formed at a lower end of the valve core assembly and is communicated with the air inlet passage (61), and an outlet is formed at side end of the valve core assembly;
a sealing ring structure, comprising a sealing ring (41) sealed in the annular mounting cavity (a), an inner ring of the sealing ring (41) is sleeved on the valve core assembly, and an outer ring of the sealing ring (41) is fixedly installed in the mounting cavity, so as to separate the annular mounting cavity (a) into a closed upper annular mounting cavity (a1) and a closed lower annular mounting cavity (a2), and the upper annular mounting cavity (a1) is communicated with an outlet of the air-guide passage (b);
a preload force structure, arranged between the valve core assembly and an inner wall of the mounting cavity, and used for providing downward preload force for the valve core assembly; and
an air outlet gap communicating with the lower annular mounting cavity (a2) can be formed in use between the lower end of the valve core assembly and an upper end of the air inlet seat (6) **characterized in that**:
the electromagnet assembly (2) comprises a coil (21) extending up and down and fixedly installed in the mounting cavity and a static armature (22) fixedly installed in an inner hole of the coil (21), when the coil (21) is electrified, the static armature (22) forms the magnetic attraction; the valve core assembly comprises a mobile armature (31) and a valve core (32), the mobile armature (31) is sandwiched between the static armature (22) and the valve core (32), an upper end of the mobile armature (31) is spaced from a lower end of the static armature (22), so as to form an electromagnet air gap (c); a lower end of the mobile armature (31) is connected with an upper end of the valve core (32), and the air outlet gap is formed between a lower end face of the valve core (32) and an upper end face of the air inlet seat (6);
the valve housing assembly (1) further comprises:
a housing (12), a lower end face of the housing (12) is penetrated with a mounting hole extending up and down, and the coil (21) is mounted in the mounting hole;
a second annular support seat (13), installed at the lower end of the housing (12), and an inner hole of the second annular support seat (13) is communicated with the mounting hole to jointly form the mounting cavity;
a valve body (14), installed in the inner hole of the second annular support seat (13) and located above the air inlet seat (6); and
the valve body (14) and the air inlet seat (6) are integrally arranged.

2. A proportional flow valve (100), comprising:
a valve housing assembly (1), formed with a mounting cavity with an opening at a lower end, an air inlet seat (6) is formed at an opening end of the mounting cavity and located at the middle part of the mounting cavity, and an air inlet passage (61) extending up and down is formed in the air inlet seat (6);
an electromagnet assembly (2), arranged at an upper end of the mounting cavity and used for providing upward magnetic attraction;
a valve core assembly, arranged in the mounting cavity and located at the middle part of the mounting cavity, an annular mounting cavity (a) is formed between the valve core assembly and an inner wall surface of the mounting cavity; driven by the magnetic attraction, the valve core assembly has an upward moving stroke; an air-guide passage (b) is formed in the valve core assembly, an inlet of the air-guide passage (b) is formed at a lower end of the valve core assembly and is communicated with the air inlet passage (61), and an outlet is formed at side end of the valve core assembly;
a sealing ring structure, comprising a sealing ring (41) sealed in the annular mounting cavity (a), an inner ring of the sealing ring (41) is sleeved on the valve core assembly, and an outer ring of the sealing ring (41) is fixedly installed in the mounting cavity, so as to separate the annular mounting cavity (a) into a closed upper annular mounting cavity (a1) and a closed lower annular mounting cavity (a2), and the upper annular mounting cavity (a1) is communicated with an outlet of the air-guide passage (b);
a preload force structure, arranged between the valve core assembly and an inner wall of the mounting cavity, and used for providing downward preload force for the valve core assembly; and
an air outlet gap communicating with the lower annular mounting cavity (a2) can be formed in use between the lower end of the valve core assembly and an upper end of the air inlet seat (6) **characterized in that**;
the electromagnet assembly (2) comprises a coil (21) extending up and down and fixedly installed in the mounting cavity and a static armature (22) fixedly installed in an inner hole of the coil (21), when the coil (21) is electrified, the static armature (22) forms the magnetic attraction; the valve core assembly comprises a mobile armature (31) and a valve core (32), the mobile armature (31) is sandwiched between the static armature (22) and the valve core (32), an upper end of the mobile armature (31) is spaced from a lower end of the static armature (22), so as to form an electromagnet air gap (c); a lower end of the mobile armature (31) is connected with an upper end of the valve core (32), and the air outlet gap is formed between a lower end face of the valve core (32) and an upper end face of the air inlet seat (6);
the lower end face of the valve core (32) is provided with a connecting hole;
a lower end face of the mobile armature (31) is provided with a third mounting hole extending up and down; and
the valve core assembly further comprises a connecting column (35), an upper end of the connecting column (35), is installed in the third mounting hole, a lower end of the connecting column (35) is inserted into the connecting hole, and the air-guide passage (b) is formed in the connecting column (35).

3. The proportional flow valve (100) of claim 1 or 2, wherein the preload force structure comprises an annular elastic sheet (51), an inner end of the annular elastic sheet (51) is fixed to the valve core assembly, and an outer end is fixed to the inner wall of the mounting cavity; an annular surface of the annular elastic sheet (51) is provided with a hollow pattern combination, the hollow pattern combination is a central symmetric structure and comprises a plurality of hollow pattern units, and the number of the hollow pattern units is an integer multiple of a natural number 2 or more.

4. The proportional flow valve (100) of claim 3, wherein a first air-guide passage segment (b1) is through installed at the lower end face of the valve core (32), and the lower end face of the mobile armature (31) is provided with a first mounting hole (311) extending up and down; a sidewall surface of the first mounting hole (311) is provided with a second air-guide passage segment (b2), the upper end of the valve core (32) is installed in the first mounting hole (311), and the first air-guide passage segment (b1) is communicated with the second air-guide passage segment (b2) to jointly form the air-guide passage (b); and/or
the mobile armature (31) is positioned at a lower end of the inner hole of the coil (21), and a non-metallic magnetic separator (7) is arranged between the upper end of the mobile armature (31) and the lower end of the static armature (22); and/or
the mobile armature (31) is at least partially positioned in the coil (21) and has a mounting segment positioned in the coil (21);
the proportional flow valve (100) further comprises a first magnetic shield sleeve (8) extending up and down, and the first magnetic shield sleeve (8) is arranged between the coil (21) and the mobile armature (31) and at least partially covers the mounting segment.

5. The proportional flow valve (100) of claim 4, wherein an upper end of the first magnetic shield sleeve (8) is positioned in the inner hole of the coil (21), and a lower end of the first magnetic shield sleeve (8) protrudes from a lower end of the coil (21);
the lower end of the static armature (22) and the upper end of the mobile armature (31) are both installed in the first magnetic shield sleeve (8), and the sidewall of the static armature (22) is in sealing abutment with an inner wall of the first magnetic shield sleeve (8).

6. The proportional flow valve (100) of claim 5, wherein the air-guide passage (b) is formed in the valve core (32) and the mobile armature (31);
an air gap is formed between a sidewall of the mobile armature (31) and an outer sidewall of the first magnetic shield sleeve (8), and the air gap communicates with the electromagnet air gap (c), the outlet of the air-guide passage (b) and the upper annular mounting cavity (a1).

7. The proportional flow valve (100) of claim 1, wherein the mounting cavity is arranged in steps to form a first annular step surface (11) arranged downwards;
the proportional flow valve (100) further comprises a first annular support seat (10) located below the first annular step surface, and the outer ring of the first annular support seat (10) is in sealing abutment with an inner sidewall of the mounting cavity, the inner ring of the first annular support seat (10) is sleeved on the valve core assembly, and a first annular mounting space is formed between an upper end face of the first annular support seat (10) and the first annular step surface, and the sealing ring (41) is clamped in the annular space.

8. The proportional flow valve (100) of claim 7, wherein an inner hole of the first annular support seat (10) is set as a tapered hole with a small top and a large bottom;
the lower end of the valve core assembly extends from a lower end of the tapered hole and movably abuts against the upper end face of the air inlet seat (6), and the lower end of the valve core assembly is arranged in steps to form a second annular step surface (33) arranged upwards;
the preload force structure comprises an annular elastic sheet (51), an inner ring of the annular elastic sheet (51) abuts against the second annular step surface (33), and an outer ring of the annular elastic sheet (51) is fixed on a lower end face of the first annular support seat (10), and the second annular step surface (33) is located above the lower end face of the first annular support seat (10).

9. The proportional flow valve (100) of claim 1, wherein an upper end sidewall of the valve body (14) is in threaded connection with an inner wall of the inner hole of the second annular support seat (13); and/or
the second annular support seat (13) comprises a support seat cylinder (131) and a second magnetic shield sleeve (132) extending upward from an upper end face of the support seat cylinder (131), the upper end face of the support seat cylinder (131) is in sealing abutment with the lower end face of the housing (12), an upper end of the second magnetic shield sleeve (132) extends into the inner hole of the coil (21), the lower end of the static armature (22) and the upper end of the mobile armature (31) are both installed in the second magnetic shield sleeve (132), and the side wall of the static armature (22) is in sealing abutment with an inner wall of the second magnetic shield sleeve (132); and/or
an upper end face of the valve body (14) is penetrated with a second mounting hole, and the second mounting hole is arranged in steps to form an abutment step surface (141) arranged upward, and the proportional flow valve (100) further comprises a first clamping assembly (10), the first clamping assembly (10) is annularly arranged and located on the abutment step surface (141); an outer ring of the first clamping assembly (10) is in sealing abutment with an inner wall surface of the second mounting hole; a first annular clamping space is formed in the first clamping assembly (10); the inner ring of the sealing ring (41) is hermetically sleeved on the valve core assembly, and the outer ring of the sealing ring (41) is clamped in the first annular clamping space.

10. The proportional flow valve (100) of claim 9, wherein the proportional flow valve (100) further comprises a transition block (101), the transition block (101) is positioned in the mounting hole and below the coil (21), and the transition block (101) is hermetically sleeved at a lower end of the second magnetic shield sleeve (132), a side wall of the transition block (101) is in sealing abutment with a side wall of the mounting hole, and a lower end face of the transition block (101) is mounted to the upper end face of the support seat cylinder (131); and/or
an inner hole of the support seat cylinder (131) is set as a tapered hole with a small top and a large bottom, and the lower end of the valve core assembly is arranged in steps to form a third annular step surface (34) arranged upwards, and the preload force structure comprises an annular elastic sheet (51), the inner ring of the annular elastic sheet (51) abuts against the third annular step surface (34), and the outer ring of the annular elastic sheet (51) is fixed on the upper end face of the valve body (14).

11. The proportional flow valve (100) of claim 2, wherein the connecting column (35) is arranged in steps to form a fourth step surface arranged upwards, and a second annular mounting space is formed between the fourth step surface and the lower end face of the mobile armature (31);
the mounting cavity is formed with a fifth step surface (15) arranged downwards, and the fifth step surface (15) is located below the fourth step surface;
the preload force structure comprises an annular elastic sheet (51), the inner ring of the annular elastic sheet (51) is clamped in the second annular mounting space, and the outer ring of the annular elastic sheet (51) abuts against the fifth step surface (15).

12. The proportional flow valve (100) of claim 11, wherein the connecting column (35) is also formed with a sixth step surface (352) arranged downwards, and a third annular mounting space is formed between the sixth step surface (352) and an upper end face of the valve core (32);
the mounting cavity is formed with a seventh annular step surface (16) arranged downwards, and the seventh annular step surface (16) is provided with a second clamping assembly (102), the second clamping assembly (102) is annularly arranged, an outer ring of the second clamping assembly (102) is in sealing abutment with the inner wall surface of the mounting cavity, and a fourth annular clamping space is formed in the second clamping assembly (102);
the inner ring of the sealing ring (41) is clamped in the third annular mounting space, and the outer ring of the sealing ring (41) is clamped in the fourth annular clamping space.
